# DEMANDE DE BREVET EUROPEEN

(11) **EP 4 806 563 A1**
(43) Date de publication de la demande: **16.09.2026**
(21) Numéro de dépôt: 26160846.7
(22) Date de dépôt: 26.02.2026
(51) Int. Cl.: B06B 1/06, A61B 8/00, H10N 30/87

(54) **METHODE DE FABRICATION D'UNE SONDE ULTRASONORE, ET SONDE ULTRASONORE CORRESPONDANTE**

(30) Priorité: 11.03.2025 FR 2502406
(71) Demandeur: Vermon, 37000 Tours (FR)
(72) Inventeur: MONTAUBAN, Emmanuel, 37000 Tours (FR)
(74) Mandataire: Cabinet Beaumont

(57) **Abrégé**

La présente description concerne une méthode de fabrication d'une sonde ultrasonore (200) comprenant :
- la formation d'une couche diélectrique (201) sur un bord d'un empilement acoustique (210) comprenant une couche piézoélectrique située sur une première portion (220A) d'un substrat d'interconnexion (220), ladite première portion étant sur une couche d'atténuation acoustique (215) et étant prolongée par une deuxième portion (220B) repliée sur un bord (215A) de la couche d'atténuation acoustique ;
- la formation d'une structure conductrice (202) de reprise de masse sur des éléments transducteurs (20) formés dans l'empilement acoustique et séparés par des encoches (214) dans l'empilement acoustique dans son épaisseur jusqu'au substrat d'interconnexion ; ladite structure conductrice s'étendant sur la couche diélectrique et étant reliée à au moins un plot (221) de contact électrique du substrat d'interconnexion ;
- la formation d'une couche de protection (204) pour recouvrir et entourer l'empilement acoustique et la structure conductrice sur la hauteur des encoches.

## Description

### Domaine technique

La présente description concerne de façon générale les sondes ultrasonores comprenant un réseau d'éléments transducteurs ultrasonores. La présente description concerne en particulier des sondes ultrasonores dont les éléments transducteurs sont des éléments transducteurs piézoélectriques. La présente description concerne notamment la fabrication de telles sondes ultrasonores.

### Technique antérieure

Certaines sondes ultrasonores comportent un réseau d'éléments transducteurs disposés les uns à côté des autres selon un arrangement linéaire (barrette), ou selon un arrangement matriciel.

Les éléments transducteurs du réseau sont de préférence adressés individuellement. Un exemple de sonde ultrasonore comprend un réseau d'éléments transducteurs dont les signaux peuvent être déphasés entre eux, ou "phased array" en anglais.

Lorsque les éléments transducteurs sont des éléments transducteurs piézoélectriques, chaque élément transducteur comprend typiquement une couche de matériau piézoélectrique, ou couche piézoélectrique, et des électrodes avant et arrière (ou électrodes supérieure ou inférieure) adaptées à appliquer à l'élément transducteur un signal électrique d'excitation et/ou à récupérer de l'élément transducteur un signal pour être transformé en signal électrique. Une des électrodes avant et arrière peut être connectée à la masse, et l'autre peut être dédiée au signal.

Ces sondes ultrasonores peuvent être utilisées pour de l'imagerie 2D, voire 3D, notamment dans des applications d'imagerie médicale, en particulier pour de l'imagerie médicale externe, c'est-à-dire sans intrusion dans un corps.

**Il** serait souhaitable de pouvoir disposer d'une sonde ultrasonore palliant au moins en partie certains des inconvénients des sondes ultrasonores connues.

**Il** serait souhaitable de pouvoir disposer d'une méthode de fabrication d'une telle sonde ultrasonore, cette méthode palliant au moins en partie certains des inconvénients des méthodes de fabrication connues.

### Résumé de l'invention

Un mode de réalisation pallie tout ou partie des inconvénients des méthodes connues de fabrication de sondes ultrasonores, et des sondes ultrasonores obtenues.

Un mode de réalisation prévoit une méthode de fabrication d'une sonde ultrasonore, la méthode comprenant :
- la formation d'une couche diélectrique sur au moins un bord d'un empilement acoustique comprenant au moins une couche piézoélectrique située sur une première portion d'un substrat d'interconnexion, ladite première portion dudit substrat d'interconnexion étant sur une couche d'atténuation acoustique et étant prolongée par une deuxième portion du substrat d'interconnexion repliée sur un bord de la couche d'atténuation acoustique ;
- la formation d'une structure conductrice de reprise de masse sur des éléments transducteurs formés dans l'empilement acoustique et séparés par des encoches provenant de la découpe de l'empilement acoustique dans toute son épaisseur, ladite découpe s'étendant au moins jusqu'au substrat d'interconnexion ; ladite structure conductrice s'étendant sur la couche diélectrique et étant reliée à au moins un plot de contact électrique du substrat d'interconnexion ;
- la formation d'une couche de protection pour recouvrir et entourer l'empilement acoustique et la structure conductrice au moins sur la hauteur des encoches.

Selon un mode de réalisation, la découpe s'étend dans la couche d'atténuation acoustique et inclut la découpe d'au moins la première portion du substrat d'interconnexion.

Selon un mode de réalisation, la couche diélectrique est configurée pour protéger le au moins un bord de l'empilement acoustique destiné à être recouvert par la structure conductrice. Par exemple, un bord de l'empilement acoustique destiné à être recouvert par la structure conductrice est aligné avec le bord de la couche d'atténuation acoustique sur lequel la deuxième portion du substrat d'interconnexion est repliée.

Selon un mode de réalisation, la formation de la structure conductrice comprend :
- la formation d'une couche métallique sur la couche diélectrique, sur et autour de l'empilement acoustique au moins jusqu'à être en contact avec le au moins un plot ; et
- la découpe de l'empilement acoustique en les éléments transducteurs séparés entre eux par les encoches, ladite découpe incluant en outre la découpe de la couche métallique.

Selon un mode de réalisation, la formation de la structure conductrice comprend :
- la découpe de l'empilement acoustique en les éléments transducteurs séparés entre eux par les encoches ; puis
- la formation d'un réseau de traces métalliques comprenant des premières traces sur chacun des éléments transducteurs, chaque première trace étant prolongée par une deuxième trace s'étendant sur la couche diélectrique et reliée à l'au moins un plot.

Selon un mode de réalisation, les deuxièmes traces sont connectées à une troisième trace connectée à l'au moins un plot.

Selon un mode de réalisation, les deuxièmes traces sont connectées directement à l'au moins un plot.

Selon un mode de réalisation, la méthode comprend en outre la formation d'une couche de blindage électromagnétique recouvrant les bords de la couche d'atténuation acoustique sur toute ou partie de sa hauteur, ainsi qu'au moins une partie du substrat d'interconnexion, la couche de blindage électromagnétique étant une couche conductrice, par exemple sous la forme d'un adhésif en cuivre.

Selon un mode de réalisation, la couche de blindage électromagnétique recouvre également le au moins un plot.

Selon un mode de réalisation, la méthode comprend en outre la formation d'une couche recouvrant et entourant la couche de protection au moins sur la hauteur des encoches de manière à former une lentille, la couche étant par exemple une couche de silicone.

Selon un mode de réalisation, la découpe de l'empilement acoustique inclut la découpe d'une première couche métallique disposée au-dessus de la couche piézoélectrique, par exemple sur l'empilement acoustique, et d'une deuxième couche métallique disposée sous la couche piézoélectrique, par exemple entre l'empilement acoustique et la couche d'atténuation acoustique, de manière à former respectivement des premières électrodes et des deuxièmes électrodes des éléments transducteurs, les premières électrodes étant reliées à l'au moins un plot par l'intermédiaire de la structure conductrice.

Un mode de réalisation prévoit une sonde ultrasonore comprenant :
- un substrat d'interconnexion comprenant au moins une première portion et une deuxième portion reliée à la première portion ;
- un empilement acoustique comprenant au moins une couche piézoélectrique située sur la première portion du substrat d'interconnexion ;
- une couche diélectrique sur au moins un bord de l'empilement acoustique ;
- une couche d'atténuation acoustique, la première portion du substrat d'interconnexion étant entre ladite couche d'atténuation acoustique et la couche piézoélectrique, et la deuxième portion du substrat d'interconnexion étant repliée sur un bord de ladite couche d'atténuation acoustique ; l'empilement acoustique étant divisé en plusieurs éléments transducteurs séparés par des encoches traversant ledit empilement acoustique dans toute son épaisseur, lesdites encoches s'étendant au moins jusqu'au substrat d'interconnexion ;
- une structure conductrice de reprise de masse reliée à chacun des éléments transducteurs, la structure conductrice s'étendant sur la couche diélectrique et étant reliée à au moins un plot de contact électrique du substrat d'interconnexion ; et
- une couche de protection recouvrant et entourant l'empilement acoustique et la structure conductrice au moins sur la hauteur des encoches.

Selon un mode de réalisation, les encoches s'étendent au travers d'au moins la première portion du substrat d'interconnexion jusque dans la couche d'atténuation acoustique.

Selon un mode de réalisation, la sonde ultrasonore comprend en outre :
- une couche de blindage électromagnétique recouvrant les bords de la couche d'atténuation acoustique sur toute ou partie de sa hauteur, ainsi qu'au moins une partie du substrat d'interconnexion ; et/ou
- une couche formant une lentille recouvrant et entourant la couche de protection au moins sur la hauteur des encoches.

Les mode de réalisation qui suivent peuvent s'appliquer à la méthode de fabrication et/ou à la sonde ultrasonore.

Selon un mode de réalisation, la couche de protection est un film polymère, par exemple un film polymère adhésif.

Selon un mode de réalisation, la couche de protection est métallisée au moins sur sa face qui est en regard de la structure conductrice.

Selon un mode de réalisation, le plot de contact électrique est positionné sur la deuxième portion du substrat d'interconnexion.

Selon un mode de réalisation, les encoches sont en air.

Selon un mode de réalisation, l'empilement acoustique comprend au moins une couche d'adaptation d'impédance sur la couche piézoélectrique.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1A est une vue de côté représentant des détails d'un exemple de sonde ultrasonore ;
la figure 1B est une vue de face de la sonde ultrasonore de la figure 1A ;
la figure 2A, la figure 2B, la figure 2C, la figure 2D, la figure 2E, la figure 2F et la figure 2G sont des vues de côté et de face illustrant des étapes successives d'une méthode de fabrication selon un mode de réalisation ;
la figure 3 représente un substrat d'interconnexion d'une sonde ultrasonore selon un mode de réalisation ;
la figure 4A, la figure 4B, la figure 4C, la figure 4D, la figure 4E et la figure 4F sont des vues de côté et de face illustrant des étapes successives d'une méthode de fabrication selon un autre mode de réalisation ;
la figure 5A représente, en vues de côté, de face et de dessus, une variante de la méthode de fabrication des figures 4A à 4F ; et
la figure 5B et la figure 5C représentent un substrat d'interconnexion d'une sonde ultrasonore selon un autre mode de réalisation.

### Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, la réalisation des éléments transducteurs des sondes ultrasonores décrites n'est pas détaillée, les modes de réalisation décrits étant compatibles avec toutes ou la plupart des structures connues de transducteurs ultrasonores. En outre, la réalisation des circuits de commande des éléments transducteurs n'est pas détaillée, les modes de réalisation décrits étant compatibles avec les circuits de commande usuels de transducteurs ultrasonores ou la réalisation des circuits de commande étant à la portée de la personne du métier à partir des indications de la présente description.

Sauf précision contraire, lorsque l'on fait référence à deux éléments connectés entre eux, cela signifie directement connectés sans éléments intermédiaires autres que des connecteurs, et lorsque l'on fait référence à deux éléments reliés (en anglais « coupled ») entre eux, cela signifie que ces deux éléments peuvent être connectés ou être reliés par l'intermédiaire d'un ou plusieurs autres éléments.

Dans la description qui suit, lorsque l'on fait référence à des qualificatifs de position absolue, tels que les termes « avant », « arrière », « haut », « bas », « gauche », « droite », etc., ou relative, tels que les termes « dessus », « dessous », « supérieur », « inférieur », etc., ou à des qualificatifs d'orientation, tels que les termes « horizontal », « vertical », etc., il est fait référence, sauf précision contraire, à l'orientation des figures.

Dans la description qui suit, lorsque l'on fait référence au terme « latéral » ou « longitudinal », il est fait référence à la direction X repérée dans les figures, et lorsque l'on fait référence au terme « transversal », il est fait référence à la direction à la direction Y, qui est perpendiculaire à la direction X. Lorsque l'on fait référence au terme « vertical », il est fait référence à la direction Z repérée dans les figures, qui est perpendiculaire aux directions X et Y.

Sauf précision contraire, les expressions « environ », « approximativement », « sensiblement », et « de l'ordre de » signifient à 10 % ou à 10° près, de préférence à 5 % ou à 5° près.

Dans la description qui suit, lorsque l'on fait référence à des éléments conducteurs, il est fait référence sauf précision contraire à des éléments électriquement conducteurs. Similairement, lorsque l'on fait référence à des éléments isolants, il est fait référence sauf précision contraire à des éléments électriquement isolants.

Dans la description qui suit, lorsque l'on fait référence à un plot, à une piste, à une trace ou à un connecteur, il est fait référence sauf précision contraire respectivement à un plot de connexion électrique, à une piste de connexion électrique, à une trace de connexion électrique ou à un connecteur électrique.

Dans la description qui suit, lorsque l'on fait référence à une sonde, il est fait référence sauf précision contraire à une sonde ultrasonore comprenant un réseau d'éléments transducteurs. En outre, lorsque l'on fait référence à des éléments transducteurs, ou à des transducteurs, il est fait référence sauf précision contraire à des éléments transducteurs piézoélectriques.

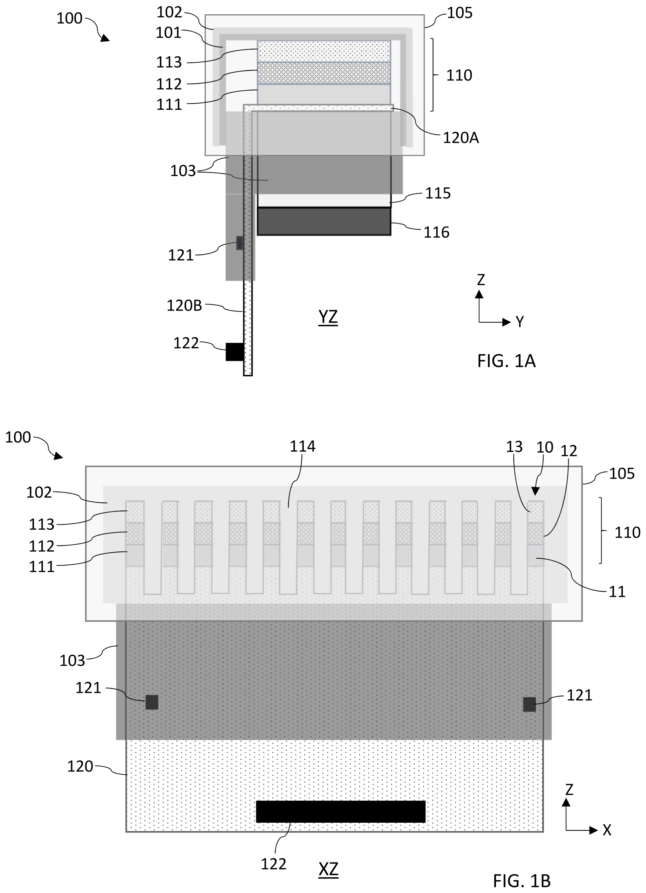

La figure 1A est une vue de côté représentant des détails d'un exemple de sonde ultrasonore 100. La figure 1B est une vue de face de la sonde ultrasonore 100 de la figure 1A. La vue de côté est représentée dans un plan YZ (plan transversal). La vue de face est représentée dans un plan XZ (plan longitudinal ou latéral).

La sonde ultrasonore 100 comprend une couche piézoélectrique 111.

La sonde ultrasonore 100 comprend en outre une ou plusieurs couches, ou lames, d'adaptation d'impédance acoustique sur la face supérieure (face avant) de la couche piézoélectrique 111 destinée à être orientée du côté d'une région à imager. Dans l'exemple des figures 1A et 1B, on a représenté deux couches d'adaptation d'impédance 112 et 113 : une couche d'adaptation d'impédance 112 sur la couche piézoélectrique 111 et une couche d'adaptation d'impédance 113 sur la couche d'adaptation d'impédance 112.

L'empilement des couches 111, 112 et 113 peut être désigné empilement acoustique 110, ou stack acoustique.

Les faces supérieure et inférieure du stack acoustique 110 sont métallisées, c'est-à-dire que le stack acoustique 110 est recouvert sur chacune de ses faces supérieure et inférieure par une couche métallique : une couche métallique sur la face supérieure (couche métallique supérieure) et une autre couche métallique sur la face inférieure (couche métallique inférieure). Les couches d'adaptation d'impédance 112 et 113 sont alors de préférence au moins partiellement conductrices.

La sonde ultrasonore 100 comprend en outre une couche d'atténuation acoustique 115, ou "backing", sous la face inférieure (face arrière) de la couche piézoélectrique 111, ainsi qu'une couche de support mécanique 116, ou raidisseur, sous la couche d'atténuation acoustique 115.

La sonde ultrasonore 100 comprend un substrat d'interconnexion 120.

Le substrat d'interconnexion 120 comprend des éléments d'interconnexion conducteurs (non représentés dans les figures 1A et 1B), généralement sous la forme de pistes d'interconnexion, ou pistes, formées dans et/ou sur un support diélectrique, généralement flexible ou souple. Les pistes sont généralement métalliques, par exemple en cuivre. Le support diélectrique est par exemple en un matériau polymère, tel qu'un polyimide.

Le substrat d'interconnexion 120 est par exemple un circuit imprimé flexible.

Le substrat d'interconnexion 120 comprend une portion 120A sensiblement horizontale située entre la couche piézoélectrique 111 et la couche de backing 115. La couche métallique inférieure est en contact avec le substrat d'interconnexion 120.

Le substrat d'interconnexion 120 comporte en outre une portion 120B reliée à la portion 120A et repliée sur un bord, ou flanc, de la couche de backing 115 et du raidisseur 116. La portion 120B peut être sensiblement verticale. La portion 120B peut se prolonger verticalement au-delà du raidisseur 116.

La sonde ultrasonore 100 comprend en outre une couche de blindage électromagnétique 103, qui est une couche conductrice, par exemple sous la forme d'un adhésif en cuivre, recouvrant les flancs de la couche de backing 115 sur toute ou partie de sa hauteur, ainsi qu'au moins une partie du substrat d'interconnexion 120. La couche de blindage électromagnétique 103 recouvre notamment des plots 121 de contact électrique du substrat d'interconnexion 120, et ainsi assure une continuité électrique avec ces plots 121.

Le stack acoustique 110 est découpé selon plusieurs plans transversaux YZ en plusieurs éléments transducteurs 10 séparés entre eux par des encoches 114, ou entailles, de l'anglais "kerfs". Cette découpe inclut la découpe de chacune des couches métalliques inférieure et supérieure en respectivement plusieurs électrodes inférieures et supérieures. Les encoches 114 s'étendent au moins jusqu'à la couche métallique inférieure de manière à isoler électriquement les électrodes inférieures de chaque élément transducteur 10 (décrit ci-dessous).

La couche piézoélectrique 111 est ainsi divisée en plusieurs éléments piézoélectriques 11. La couche d'adaptation d'impédance acoustique 112 est divisée en plusieurs éléments 12, et la couche d'adaptation d'impédance acoustique 113 est divisée en plusieurs éléments 13. Chaque empilement des éléments 11, 12 et 13 forme un élément transducteur 10.

Les électrodes inférieures sont en contact avec des pistes du substrat d'interconnexion 120 et forment des électrodes de signal des différents éléments transducteurs 10. Les électrodes supérieures forment des électrodes de masse des éléments transducteurs 10.

Les kerfs 114 sont de préférence laissés en air et sont protégés par un empilement de couches 101 et 102 qui recouvrent et entourent l'empilement acoustique 110 au moins sur la hauteur des kerfs 114. La couche 101 est un film conducteur, de préférence adhésif, par exemple un film conducteur adhésif de la marque Arclad^{®}. La couche 102 est un film polymère, par exemple en polyéthylène, qui recouvre le film conducteur 101. Le film polymère 102 est de préférence métallisé au moins sur sa face inférieure qui est en contact avec le film conducteur 101.

Le film conducteur 101 a une fonction de blindage électromagnétique.

**En** outre, le film conducteur 101 vient au contact de la couche de blindage électromagnétique 103, et est ainsi relié aux plots 121 et au substrat d'interconnexion 120. Il présente ainsi également une fonction de reprise de masse des éléments transducteurs 10 via les électrodes supérieures de ces éléments transducteurs.

La sonde ultrasonore 100 comprend en outre une couche de protection 105 au moins sur la face supérieure (face avant) de l'empilement acoustique 110, en particulier sur les couches d'adaptation d'impédance 112, 113. Cette couche de protection 105 est destinée à être en contact avec la région à imager. La couche de protection 105 est constituée d'un matériau permettant la propagation des ondes ultrasonore, par exemple un matériau biocompatible, par exemple du silicone. La face supérieure de la couche de protection 105 peut être plane ou former une lentille acoustique, par exemple être convexe, apte à focaliser les ondes ultrasonores. Cette couche de protection 105 peut être désignée dans la suite de la description comme une couche de silicone ou une lentille silicone, sans que cela soit restrictif de sa forme et de son matériau. Dans l'exemple représenté, la couche de silicone 105 recouvre et entoure l'empilement des couches 101 et 102 au moins sur la hauteur des kerfs 114, bien que cela ne soit pas limitatif.

La couche de silicone 105 peut être réalisée par moulage. Les couches 101 et 102 protègent avantageusement les kerfs en air 114 lors de ce procédé de moulage.

Les couches 102, 103 et 105 ont été représentées en transparence pour visualiser les éléments qui sont dessous.

Le substrat d'interconnexion 120 inclut en outre un connecteur 122 qui n'est pas recouvert par les couches 101, 102, 103 et 105. Le connecteur 122 peut être utilisé pour relier chacune des pistes du substrat d'interconnexion 120 à un autre élément de connexion électrique, par exemple à un autre substrat d'interconnexion, un circuit imprimé rigide ou flexible, un câble, un faisceau de câbles, ou plus généralement tout élément permettant de relier les électrodes des éléments transducteurs 10 à des circuits électroniques externes ou internes à la sonde ultrasonore 100.

Un inconvénient à ce type de sonde ultrasonore 100 est que, lorsque la lentille est formée par moulage de la couche de silicone 105, une force de rétractation du silicone lors du rétreint post-polymérisation peut causer le détachement des films conducteur 101 et polymère 102, créant des défauts de contact avec l'empilement acoustique 110 et/ou sur la couche de blindage électromagnétique 103, ce qui affecte négativement la reprise de masse de la sonde ultrasonore 100, et ainsi réduit ses performances.

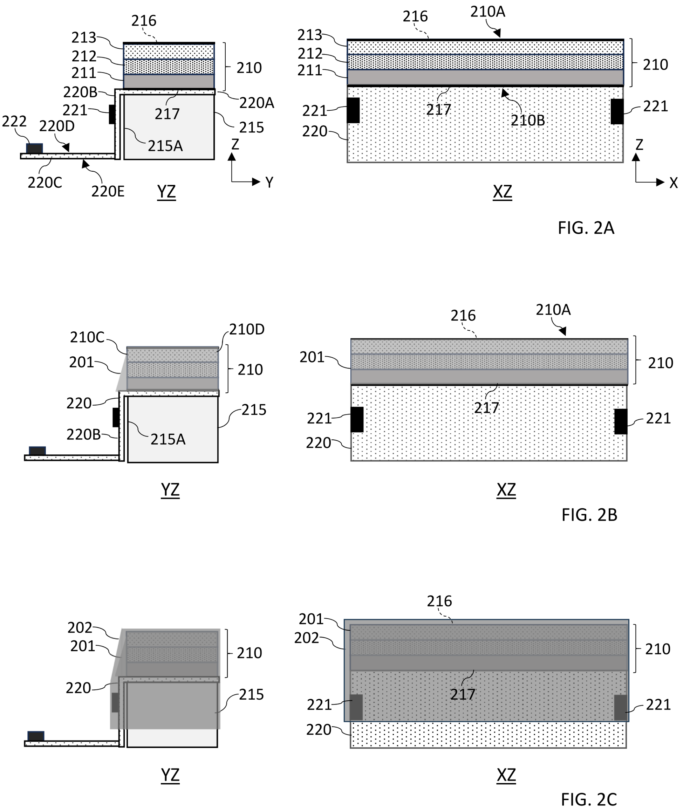

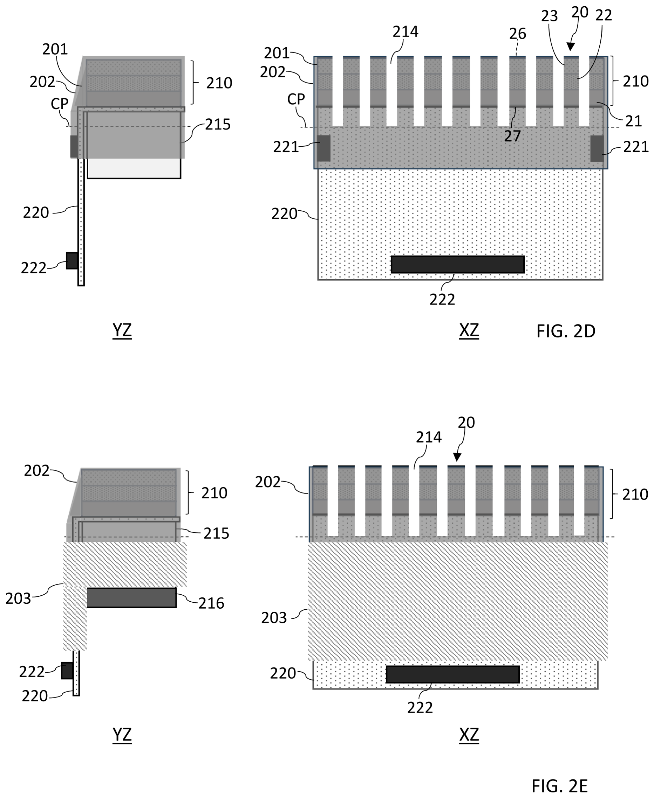

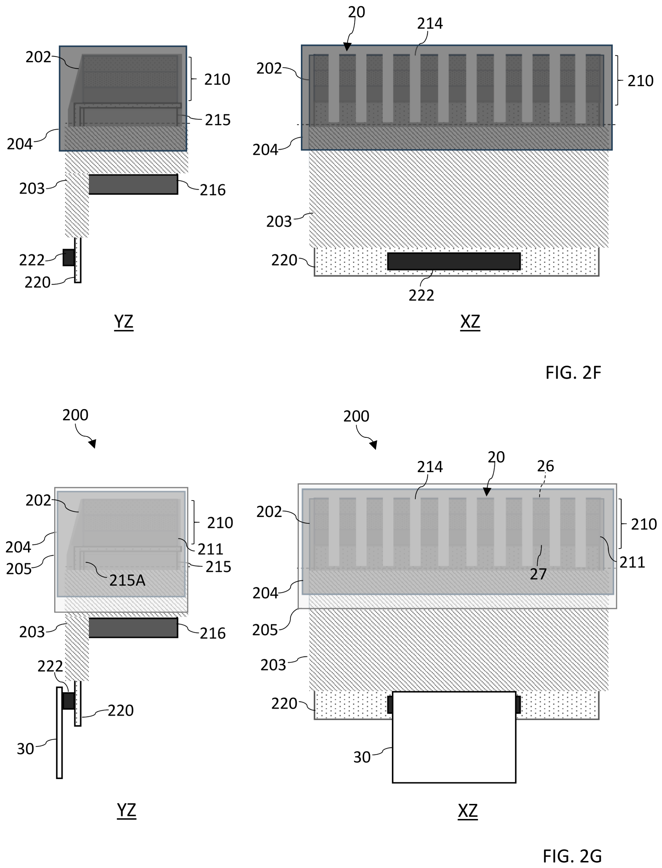

La figure 2A, la figure 2B, la figure 2C, la figure 2D, la figure 2E, la figure 2F et la figure 2G sont des vues de côté et de face illustrant des étapes successives d'une méthode de fabrication selon un mode de réalisation. Les vues de côté sont représentées dans un plan YZ (plan transversal). Les vues de face sont représentées dans un plan XZ (plan longitudinal ou latéral).

La figure 2A représente une structure de départ comprenant un empilement acoustique 210, similaire à l'empilement acoustique 110 des figures 1A et 1B, comprenant une couche piézoélectrique 211, et une ou plusieurs couches, ou lames, d'adaptation d'impédance acoustique sur la face supérieure (face avant) de la couche piézoélectrique 211 destinée à être orientée du côté d'une région à imager.

Dans l'exemple de la figure 2A, on a représenté deux couches d'adaptation d'impédance acoustique 212 et 213 : une couche d'adaptation d'impédance acoustique 212 sur la couche piézoélectrique 211 et une autre couche d'adaptation d'impédance acoustique 213 sur la couche d'adaptation d'impédance acoustique 212. En variante, il pourrait n'y avoir une seule couche d'adaptation d'impédance (par exemple pas de couche d'adaptation d'impédance 213), voire plus de deux couches d'adaptation d'impédance.

Au moins la face inférieure 210B de l'empilement acoustique 210 est métallisée, c'est-à-dire que l'empilement acoustique 210 est recouvert au moins sur sa face inférieure 210B par une couche métallique inférieure 217.

Les couches d'adaptation d'impédance 212 et 213 sont par exemple au moins partiellement conductrices. Une couche métallique supérieure 216 peut alors être prévue sur la couche d'adaptation d'impédance 213.

Selon une variante, la couche d'adaptation d'impédance 212 pourrait être conductrice, et la couche d'adaptation d'impédance 213 ne pas être conductrice, une couche métallique supérieure 216 pourrait alors être prévue entre les deux couches d'adaptation d'impédance 212 et 213.

Selon une autre variante, les deux couches d'adaptation d'impédance 212 et 213 pourraient ne pas être conductrices, une couche métallique supérieure 216 pourrait alors être prévue entre la couche piézoélectrique 211 et la couche d'adaptation d'impédance 212, par exemple sur la face supérieure de la couche piézoélectrique 211.

Les étapes suivantes de la méthode décrites ci-après en lien avec les figures 2B à 2D sont alors adaptées pour que la reprise de masse puisse être faite sur la couche métallique supérieure 216, quel que soit son emplacement.

**En** particulier, on prévoit de préférence que la couche d'isolation diélectrique 201 décrite plus après en lien avec la figure 2B, s'étende verticalement depuis le substrat d'interconnexion 220 jusqu'à la face inférieure de la couche métallique supérieure 216. Ensuite, on prévoit de préférence que l'étape de métallisation décrite en lien avec la figure 2C forme une couche métallique 202 qui vient en contact avec la couche métallique supérieure 216, et peut venir en contact avec la (ou les) couche(s) d'adaptation non isolée(s).

Un substrat d'interconnexion 220 est assemblé sous la face inférieure 210B de l'empilement acoustique 210, sous la couche piézoélectrique 211, de préférence en contact avec la couche métallique inférieure 217. Le substrat d'interconnexion 220 s'étend également au-dessus d'une couche d'atténuation acoustique 215, ou couche de backing, et sur un bord 215A, ou flanc 215A, de cette couche d'atténuation acoustique 215.

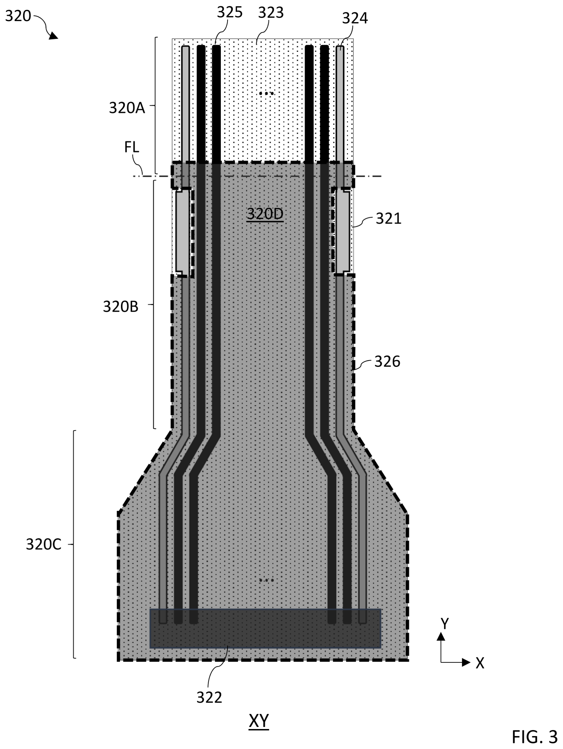

Le substrat d'interconnexion 220 comprend des éléments d'interconnexion conducteurs (non représentés dans les figures 2A à 2G mais visibles sur la figure 3 décrite plus après), généralement sous la forme de pistes d'interconnexion, ou pistes, formées dans et/ou sur un support diélectrique, généralement flexible ou souple. Les pistes sont généralement métalliques, par exemple en cuivre. Le support diélectrique est par exemple en un matériau polymère, tel qu'un polyimide. Le substrat d'interconnexion 220 est par exemple un circuit imprimé flexible.

Les pistes d'interconnexion peuvent être positionnées sur une seule face du substrat d'interconnexion. Selon une variante, les pistes d'interconnexion peuvent être positionnées sur les deux faces du substrat d'interconnexion. Par exemple, chaque piste d'interconnexion ou portion de piste d'interconnexion en face avant du substrat d'interconnexion peut se prolonger en face arrière du substrat d'interconnexion par l'intermédiaire d'un via conducteur. Selon une autre variante, le substrat d'interconnexion peut être un substrat multicouches, tel qu'un flexible multicouches, et prolonger des pistes d'interconnexion disposées en face avant du substrat d'interconnexion sur plusieurs plans, ou niveaux, séparés par une couche diélectrique, une piste d'interconnexion d'un niveau pouvant être connectée à une autre piste d'interconnexion d'un autre niveau par un via conducteur.

Le substrat d'interconnexion 220 comprend :
- une portion 220A sensiblement horizontale entre la couche piézoélectrique 211, en particulier entre la couche métallique inférieure 217, et la couche d'atténuation acoustique 215 ;
- une portion 220B sensiblement verticale reliée à la portion 220A et repliée contre le flanc 215A de la couche d'atténuation acoustique 215 ; et
- une portion 220C reliée à la portion 220B et qui peut être initialement tout ou partie sensiblement horizontale.

Le substrat d'interconnexion 220 comprend, sur la portion 220B et sur sa face avant 220D, des plots 221 de contact électrique. La face avant 220D du substrat d'interconnexion est opposée à la face arrière 220E qui est en regard de la couche d'atténuation acoustique 215. Les plots 221 peuvent être désignés plots de reprise de masse.

Le substrat d'interconnexion 220 comprend en outre, sur la portion 220C et sur sa face avant 220D, un connecteur 222. Le connecteur 222 peut être utilisé pour relier tout ou partie des pistes du substrat d'interconnexion 220 à un autre élément de connexion électrique 30 (visible sur la figure 2G), par exemple à un autre substrat d'interconnexion, un circuit imprimé rigide ou flexible, une carte électronique, un câble ou un faisceau de câbles, plus généralement tout élément permettant de relier les électrodes 26, 27 des éléments transducteurs 20 (visibles sur la figure 2D décrite plus après) à des circuits électroniques externes ou internes à la sonde ultrasonore 200 (visible sur la figure 2G décrite plus après).

La figure 2B représente une structure obtenue à l'issue de l'isolation de bords de l'empilement acoustique 210. Pour réaliser cette isolation, une couche de matériau diélectrique 201 (couche diélectrique) est formée au moins sur certains bords de l'empilement acoustique 210.

La couche diélectrique 201 n'est pas formée sur la face supérieure 210A de l'empilement acoustique 210.

La couche diélectrique 201 est formée au moins sur le bord latéral 210C (dans le plan XZ) de l'empilement acoustique 210, qui est le bord latéral positionné au-dessus du flanc 215A de la couche d'atténuation acoustique 215 (flanc sur lequel est repliée la portion 220B du substrat d'interconnexion 220).

Selon la technique de métallisation mise en œuvre dans l'étape de métallisation qui suit, par exemple en cas de dépôt d'une couche métallique 202 du type "pleine plaque" comme décrit plus après dans la figure 2C, il peut être nécessaire de former la couche diélectrique 201 sur le bord latéral 210D opposé au bord latéral 210C de l'empilement acoustique 210.

En outre, la couche diélectrique 201 peut être formée sur l'un ou les deux bords transversaux (dans le plan YZ). Par exemple, la couche diélectrique 201 peut faire le tour de l'empilement acoustique 210. Mais ceci peut ne pas être nécessaire. En effet, les bords transversaux n'ont pas nécessairement besoin d'être protégés dans la mesure où les deux éléments transducteurs 20 qui sont aux deux extrémités du réseau peuvent être rendus inactifs, leurs électrodes inférieures 27 étant par exemple reliées à des pistes conductrices 324 qui sont à des bords du substrat d'interconnexion 320, comme décrit plus après en lien avec la figure 3.

La couche diélectrique 201 permet d'éviter un court-circuit entre les électrodes inférieures 27 des éléments transducteurs 20 (décrits plus après en lien avec la figure 2D). En effet, sans protection, tous les bords latéraux de l'empilement acoustique 210 seraient métallisés, ce qui reviendrait au mieux à diminuer le champ électrique entre les électrodes inférieure 27 et supérieure 26 des éléments transducteurs 20, puisque cela créerait des électrodes latérales notamment sur le matériau piézoélectrique 211, et au pire à court-circuiter les électrodes inférieure 27 et supérieure 26.

Le matériau diélectrique de la couche diélectrique 201 peut être une résine, par exemple une résine époxy, ou un silicone, ou tout autre matériau adapté à polymériser et durcir. La formation de la couche diélectrique 201 peut être réalisée à l'aide d'un "dispenser", par exemple une seringue, adapté pour déposer le matériau diélectrique de manière calibrée, et qui est déplacé le long des bords à protéger de l'empilement acoustique 210. **En** variante, la formation de la couche diélectrique 201 peut être réalisée par pulvérisation sur les bords à protéger de l'empilement acoustique 210. Ces exemples de matériaux, ainsi que de technique de dépôt, ne sont pas limitatifs. **En** variante, la couche diélectrique 201 peut être formée par une technique d'impression 3D, en utilisant tout matériau diélectrique adapté.

La figure 2C représente une structure obtenue à l'issue de la formation d'une couche métallique 202 sur la structure de la figure 2B (étape de métallisation), en particulier sur la couche diélectrique 201, sur et autour de l'empilement acoustique 210, autour de la couche d'atténuation acoustique 215 sur tout ou partie de sa hauteur, et sur la portion 220B du substrat d'interconnexion 220 au moins jusqu'à être en contact avec les plots 221.

La couche métallique 202 permet d'établir une liaison électrique entre la face supérieure 210A de l'empilement acoustique 210 et les plots 221, pour une reprise de masse via la couche métallique supérieure 216 qui formera les électrodes supérieures 26 des éléments transducteurs 20 décrits plus après. La couche métallique 202 peut être désignée couche de reprise de masse.

Comme indiqué plus avant, ceci couvre le cas où les couches d'adaptation d'impédance 212 et 213 sont au moins partiellement conductrices, et la couche métallique supérieure 216 est sur la couche d'adaptation d'impédance 213. Plus généralement, la couche métallique supérieure 216 est positionnée différemment selon que la couche d'adaptation d'impédance 212 est conductrice ou non, et/ou la couche d'adaptation d'impédance 213 est conductrice ou non, la couche métallique 202 étant formée pour venir contact avec la couche métallique supérieure 216 quel que soit son positionnement.

La couche métallique 202 peut comprendre un empilement de couches, par exemple une couche d'accroche sur laquelle est positionnée une couche de métallisation. La couche d'accroche peut comprendre du titane (Ti), du chrome (Cr). La couche de métallisation peut comprendre de l'or (Au), du cuivre (Cu), ou de l'aluminium (Al). Par exemple, la couche métallique 202 est une couche titane et or (Ti/Au). Par exemple, la couche métallique 202 a une épaisseur comprise entre environ 200 nm et 200 µm.

La formation de la couche métallique 202 peut être réalisée par une technique de dépôt PVD (de l'anglais "physical vapor deposition", dépôt physique en phase vapeur), ou toute autre technique de dépôt adaptée.

La figure 2D représente une structure obtenue à l'issue d'une étape de découpe de l'empilement acoustique 210 selon plusieurs plans transversaux YZ en plusieurs éléments transducteurs 20 séparés entre eux par des encoches 214, ou entailles, de l'anglais "kerfs". Cette découpe inclut la découpe de la couche métallique 202. Cette découpe inclut aussi la découpe de chacune des couches métalliques supérieure 216 et inférieure 217 en respectivement plusieurs électrodes supérieures 26 et inférieures 27. La découpe, et ainsi les encoches 214, s'étendent de préférence au travers du substrat d'interconnexion 220 jusqu'à un plan de découpe CP qui se situe dans la couche de backing 215. Par exemple, la largeur des encoches 214 est comprise entre 1 µm et plusieurs centaines de micromètres, par exemple égale à environ 20 µm.

Le plan de découpe CP est de préférence situé au-dessus du niveau des plots 221.

La couche piézoélectrique 211 est ainsi divisée en plusieurs éléments piézoélectriques 21. La couche d'adaptation d'impédance acoustique 212 est divisée en plusieurs éléments 22, et la couche d'adaptation d'impédance acoustique 213 est divisée en plusieurs éléments 23. Chaque empilement des éléments 21, 22 et 23 forme un élément transducteur 20.

Les électrodes inférieures 27 sont reliées à, par exemple sont en contact avec, certaines des pistes du substrat d'interconnexion 220 et forment des électrodes de signal des éléments transducteurs 20. Les électrodes supérieures 26 forment des électrodes de masse des éléments transducteurs 20.

De plus, la portion 220C du substrat d'interconnexion 220 peut alors être complètement dépliée pour être dans l'axe de la portion 220B, voire déformée pour se rapprocher du contour de la couche de backing 215 et du raidisseur 216 (décrit ci-dessous).

La figure 2E représente une structure obtenue à l'issue d'étapes, optionnelles, de formation d'une couche de support mécanique 216, ou raidisseur, sous la couche de backing 215, et d'une couche de blindage électromagnétique 203 recouvrant les bords, ou flancs, de la couche de backing 215 sur toute ou partie de sa hauteur, ainsi qu'au moins une partie du substrat d'interconnexion 220. La couche de blindage électromagnétique 203 est une couche conductrice, par exemple sous la forme d'un adhésif en cuivre. Selon un mode de réalisation, la couche de blindage électromagnétique 203 recouvre les plots 221. De préférence, la couche de blindage électromagnétique 203 ne recouvre pas le connecteur 222.

La figure 2F représente une structure obtenue à l'issue d'une étape de formation d'une couche de protection 204 qui recouvre et entoure l'empilement acoustique 210 au moins sur la hauteur des kerfs 214.

Les kerfs 214 sont de préférence laissés en air et sont protégés par cette couche 204.

La couche de protection 204 n'est pas nécessairement conductrice, la reprise de masse étant déjà assurée par la couche métallique 202.

La couche de protection 204 est par exemple un film polymère adhésif. La couche de protection 204 est par exemple un film polymère adhésif de la marque Arclad^{®}.

La couche de protection 204 peut être métallisée au moins sur la face inférieure, c'est-à-dire la face en regard de la couche métallique 202.

La figure 2G représente une structure obtenue à l'issue d'une étape de formation d'une couche 205 en un matériau apte à propager les ondes ultrasonores, de préférence biocompatible, par exemple un polymère, par exemple une couche de silicone, sur la structure de la figure 2F. La face supérieure de la couche de protection 205 peut être plane ou former une lentille acoustique, par exemple être convexe, apte à focaliser les ondes ultrasonores.

Cette couche 205 peut être désignée dans la suite de la description comme une couche de silicone ou une lentille silicone, sans que cela soit restrictif de sa forme et de son matériau.

La couche de silicone 205 recouvre et entoure la couche de protection 204 et peut s'étendre verticalement jusqu'à la couche de support mécanique 216. La couche de silicone 205 peut être réalisée par moulage.

Les couches 201, 202, 204, 205 ont été représentées en transparence pour visualiser les éléments qui sont dessous.

La figure 2G illustre une sonde ultrasonore 200 qui se distingue de la sonde ultrasonore 100 des figures 1A et 1B principalement en ce que, dans la sonde ultrasonore 100, le film conducteur 101 a une double fonction de blindage électromagnétique et de reprise de masse, tandis que, dans la sonde ultrasonore 200 de la figure 2G, la reprise de masse est assurée par la couche métallique 202. La couche de protection 204 peut être métallisée pour former également une couche de blindage électromagnétique, mais ceci est optionnel.

De plus, dans la sonde ultrasonore 100, le film conducteur 101 est déposé après la découpe de l'empilement acoustique 110, tandis que dans la sonde ultrasonore 200, la couche métallique 202 est déposée avant la découpe de l'empilement acoustique 210, ce qui améliore l'adhésion du métal sur l'empilement acoustique 210. Ensuite, lorsque la couche de protection 204 puis la couche de silicone 205 sont déposées, **il** y a moins de risque d'arracher la couche métallique 202 du dessus de l'empilement acoustique 210 et des plots 221, réduisant ainsi le risque de défaut de reprise de masse, et de diminution de la performance de la sonde ultrasonore 200.

La figure 3 représente un substrat d'interconnexion 320 d'une sonde ultrasonore selon un mode de réalisation.

Par exemple, le substrat d'interconnexion 320 peut former ou remplacer le substrat d'interconnexion 220 de la sonde ultrasonore 200 de la figure 2G. Mis à part les références du substrat d'interconnexion, on reprend ci-dessous les références de la sonde ultrasonore 200 de la figure 2G.

Le substrat d'interconnexion 320 est représenté en position complètement dépliée (à plat).

Le substrat d'interconnexion 320 comprend :
- une portion 320A destinée à être positionnée entre la couche piézoélectrique 211, en particulier entre la couche métallique inférieure 217, et la couche d'atténuation acoustique 215 ;
- une portion 320B reliée à la portion 320A et destinée à être repliée contre le flanc 215A de la couche d'atténuation acoustique 215 : cette portion 320B supporte les plots 321 de contact électrique en face avant 320D du substrat d'interconnexion 320 ; et
- une portion 320C reliée à la portion 320B dans l'axe de la portion 320B : cette portion 320C supporte le connecteur 322 en face avant 320D du substrat d'interconnexion 320.

On a représenté une ligne de pliage **FL** entre la portion 320A et la portion 320B.

On a représenté la portion 320C qui s'élargit dans la direction X par rapport à la portion 320B, mais ceci n'est pas limitatif et la portion 320C pourrait avoir la même largeur que la portion 320B. Plus généralement, les différentes portions du substrat d'interconnexion 320 pourraient avoir toutes autres dimensions adaptées.

Le substrat d'interconnexion 320 comprend un support diélectrique 323 dans et/ou sur lequel s'étendent des pistes métalliques 324, 325.

Les pistes métalliques 324 sont localisées de préférence à proximité des bords du substrat d'interconnexion 320, et sont connectées aux plots 321. Les pistes métalliques 324 sont dédiées à la reprise de masse, en étant reliées aux électrodes supérieures 26 des éléments transducteurs 20 via la couche métallique 202 et les plots 221.

Les pistes métalliques 325 sont localisées au centre du substrat d'interconnexion 320, de préférence entre les deux pistes métalliques 324. Les pistes métalliques 325 sont dédiées aux signaux des éléments transducteurs 20 et sont reliées aux électrodes inférieures 27 des éléments transducteurs 20. Toutes les pistes métalliques 325 n'ont pas été représentées pour ne pas alourdir la figure 3, mais il peut y en avoir plus de quatre (symbolisé par le signe "..."), par exemple de l'ordre d'une centaine de pistes, par exemple 128 pistes ou 256 pistes.

Les pistes métalliques 324 et 325 s'étendent jusqu'au connecteur 322, auquel elles sont reliées.

Le substrat d'interconnexion 320 est partiellement recouvert, sur sa face avant 320D, d'une couche de protection 326 isolante (délimitée par des pointillés), par exemple un polymère. La couche de protection 326 laisse découverte presque toute la portion 320A pour que les pistes métalliques 325 puissent être reliées aux, par exemple être en contact avec les, électrodes inférieures 27 des éléments transducteurs 20. La couche de protection 326 laisse découverts les plots 321 pour que la couche de reprise de masse 201 puisse contacter ces plots 321.

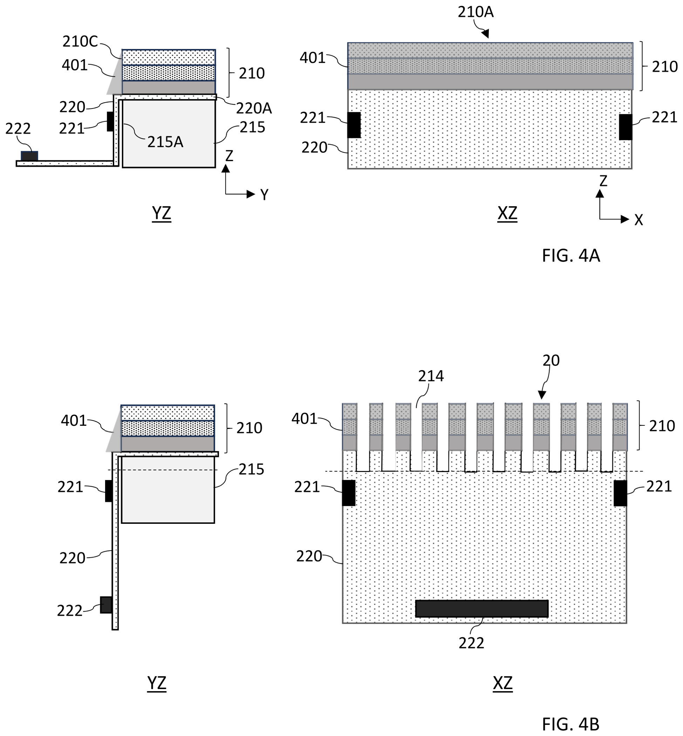

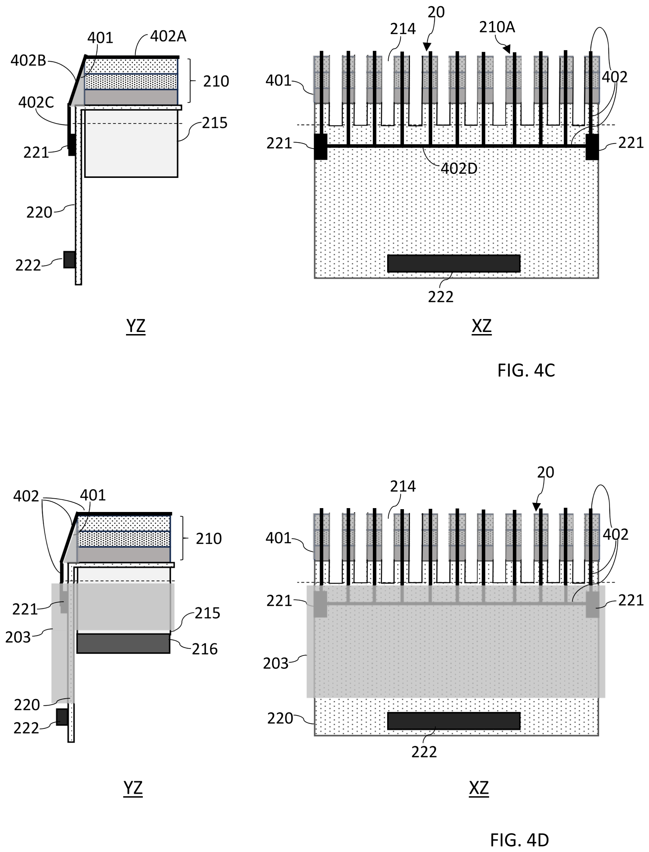

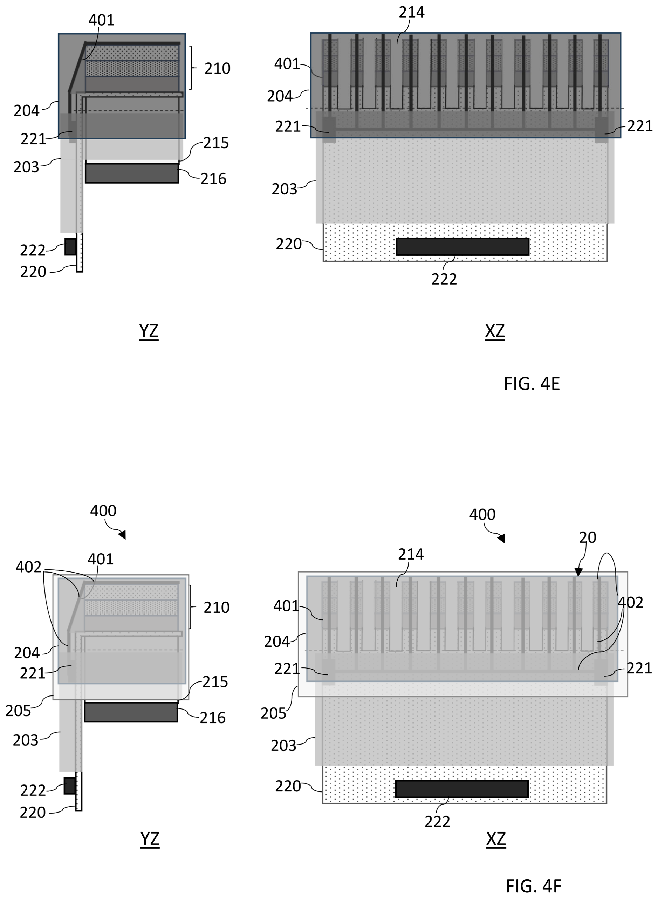

La figure 4A, la figure 4B, la figure 4C, la figure 4D, la figure 4E et la figure 4F sont des vues de côté et de face illustrant des étapes successives d'une méthode de fabrication selon un autre mode de réalisation. Les vues de côté sont représentées dans un plan YZ (plan transversal). Les vues de face sont représentées dans un plan XZ (plan longitudinal ou latéral).

La méthode de fabrication des figures 4A à 4F comprend de nombreuses étapes communes avec la méthode de fabrication des figures 2A à 2G, ces étapes communes ne seront pas détaillées à nouveau dans ce qui suit. De plus, la sonde ultrasonore 400 obtenue, visible en figure 4F, présente de nombreux éléments communs avec la sonde ultrasonore 200 de la figure 2G, ces éléments communs ne seront pas détaillés à nouveau dans ce qui suit, et conserveront les mêmes références.

La méthode de fabrication des figures 4A à 4F se distingue de la méthode de fabrication des figures 2A à 2G principalement en ce que :
- la couche diélectrique 401 n'est formée que sur le bord latéral 210C de l'empilement acoustique 210, comme représenté dans la figure 4A ;
- la découpe de l'empilement acoustique 210 en éléments transducteurs 20 est réalisée après le dépôt de la couche diélectrique 401 mais avant l'étape de métallisation, comme représenté dans la figure 4B ;
- au lieu d'une couche métallique, la reprise de masse est réalisée par la formation d'un réseau 402 de traces métalliques sur l'empilement acoustique 210 après la découpe de celui-ci, comme représenté dans la figure 4C.

Le réseau 402 de traces métalliques comprend des traces supérieures 402A formées chacune sur un des éléments transducteurs 20, les traces supérieures 402A sont prolongées par des traces intermédiaires 402B qui s'étendent sur la couche diélectrique 401, et les traces intermédiaires 402B sont prolongées par des traces inférieures 402C qui s'étendent au moins jusqu'au niveau des plots 221. Les deux plots 221 sont reliés entre eux par une autre trace métallique 402D qui relie également entre elles les traces inférieures 402C.

Les traces métalliques 402 peuvent être réalisées par une technique d'impression 3D, par exemple une technique d'impression par jet d'aérosol (en anglais "aerosol jet printing"), une technique de jet de liant métallique (en anglais, "metal binder jetting") ou toute autre technique adaptée à réaliser des traces métalliques.

Au lieu d'une couche métallique telle que la couche métallique 202 de la figure 2C, l'avantage de former des traces métalliques 402 est que cela permet de réaliser localement et plus précisément la reprise de masse des éléments transducteurs 20, et ce, en ayant moins de bords de l'empilement acoustique 210 à isoler électriquement, à la différence de la couche isolante 201 de la figure 2B.

A l'inverse, le mode de réalisation des figures 2B à 2D a l'avantage d'être plus facile en termes de procédé car le dépôt métallique peut être en pleine plaque, ou collectif, au lieu d'être sélectif. La personne du métier pourra ainsi choisir une technique de métallisation du type soustractive selon les figures 2B à 2D, ou une technique de métallisation du type additive selon les figures 4A à 4C.

Les étapes représentées dans les figures 4D, 4E et 4F correspondent sensiblement aux étapes représentées respectivement dans les figures 2E, 2F et 2G, c'est-à-dire :
- la formation de la couche de support mécanique 216 (raidisseur) sous la couche d'atténuation acoustique 215, et de la couche de blindage électromagnétique 203 ;
- la formation de la couche de protection 204 ; et
- la formation de la couche de silicone 205.

La figure 4F illustre une sonde ultrasonore 400 qui se distingue de la sonde ultrasonore 200 de la figure 2G principalement en ce que, dans la sonde ultrasonore 400, la reprise de masse est assurée par les traces métalliques 402 au lieu de la couche métallique 202. Dans la sonde ultrasonore 400, la découpe de l'empilement acoustique 210 est réalisée avant la formation des traces métalliques 402, mais le procédé de dépôt des traces métalliques 402 permet d'obtenir une bonne adhésion du métal sur les éléments transducteurs 20, similaire à celle de la sonde ultrasonore 200. Ensuite, lorsque la couche de protection 204 puis la couche de silicone 205 sont déposées, il y a moins de risque d'arracher les traces métalliques 402 du dessus de l'empilement acoustique 210 et des plots 221, que pour la sonde ultrasonore 100 des figures 1A et 1B, réduisant ainsi le risque de défaut de reprise de masse.

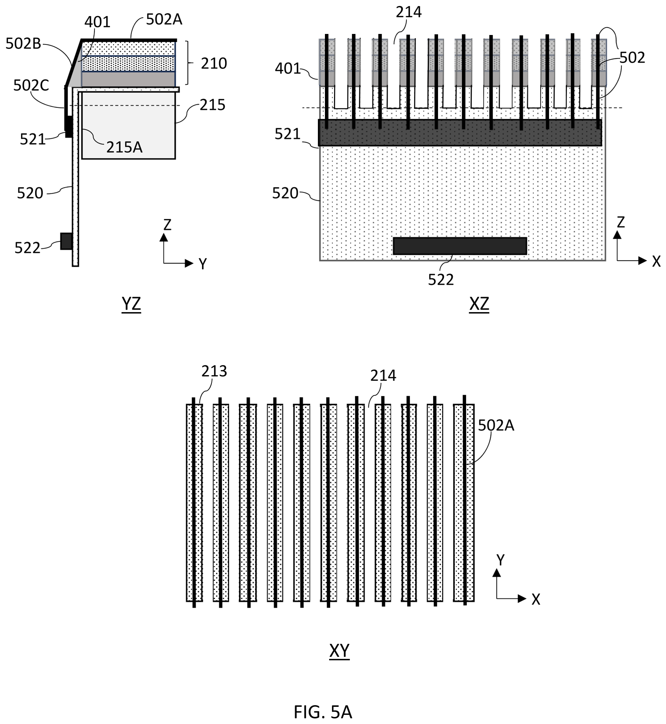

La figure 5A représente, en vues de côté, de face et de dessus, une variante de la méthode de fabrication des figures 4A à 4F.

La variante de la figure 5A comprend de nombreuses étapes communes avec la méthode de fabrication des figures 4A à 4F, ces étapes communes ne seront pas décrites à nouveau dans ce qui suit.

La variante de la figure 5A se distingue de la méthode de fabrication des figures 4A à 4F principalement en ce que le substrat d'interconnexion 520 comprend un seul plot 521 au lieu de deux plots 221 pour la reprise de masse, et en ce que le réseau 502 de traces métalliques comprend les traces supérieures 502A sur chacun des éléments transducteurs 20, les traces intermédiaires 502B sur la couche diélectrique 401, et les traces inférieures 502C qui s'étendent jusqu'au plot 521 de reprise de masse. Il n'est pas nécessaire que le réseau 502 de traces métalliques inclut la trace métallique 402D de la figure 4C.

La sonde ultrasonore (non représentée) qui sera obtenue à l'issue des étapes suivantes, similaires aux étapes des figures 4D à 4F, se distinguera par les mêmes caractéristiques.

A noter que la vue de dessus de la figure 5A pourrait représenter la vue de dessus de la figure 4C.

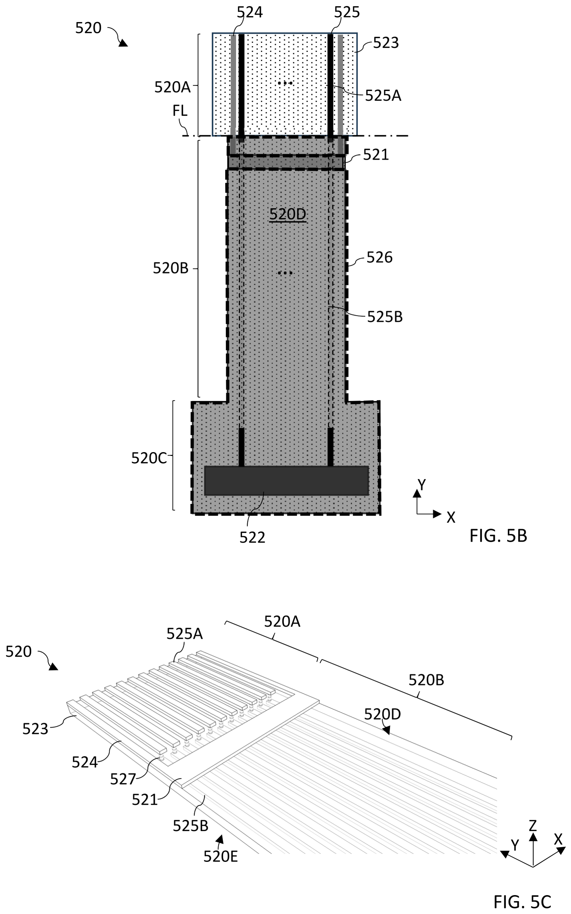

La figure 5B et la figure 5C représentent un substrat d'interconnexion 520 d'une sonde ultrasonore selon un autre mode de réalisation.

Par exemple, le substrat d'interconnexion 520 correspond au, ou remplace le, substrat d'interconnexion de la figure 5A.

Le substrat d'interconnexion 520 est représenté en position complètement dépliée (à plat). La figure 5B est une vue de dessus, et la figure 5C est une vue en trois dimensions, dans laquelle le support diélectrique 523 est représenté en transparence pour pouvoir visualiser les éléments qui autrement seraient cachés dessous.

Similairement au substrat d'interconnexion 320 de la figure 3, le substrat d'interconnexion 520 des figures 5B et 5C comprend :
- une portion 520A destinée à être positionnée entre la couche piézoélectrique, en particulier entre la couche métallique inférieure, et la couche d'atténuation acoustique 215 ;
- une portion 520B reliée à la portion 520A et destinée à être repliée contre le flanc 215A de la couche d'atténuation acoustique 215 : cette portion 520B supporte le plot 521 de contact électrique en face avant 520D du substrat d'interconnexion 520 ;
- une portion 520C reliée à la portion 520B et dans l'axe de la portion 520B : cette portion 520C supporte le connecteur 522 en face avant 520D du substrat d'interconnexion 520.

On a représenté une ligne de pliage **FL** entre la portion 520A et la portion 520B.

On a représenté la portion 520C qui est plus large dans la direction X par rapport à la portion 520B, mais ceci n'est pas limitatif et la portion 520C pourrait avoir la même largeur que la portion 520B. Plus généralement, les différentes portions du substrat d'interconnexion 520 pourraient avoir toutes autres dimensions adaptées.

Similairement au substrat d'interconnexion 320 de la figure 3, le substrat d'interconnexion 520 des figures 5B et 5C comprend un support diélectrique 523 dans et/ou sur lequel s'étendent des pistes métalliques 524, 525. Les pistes métalliques 524 sont de préférence à proximité des bords du substrat d'interconnexion 520, et sont connectées au plot 521. Les pistes métalliques 524 sont dédiées à la reprise de masse, en étant reliées aux électrodes supérieures 26 via les traces métalliques 502 et le plot 521. Les pistes métalliques 525 sont au centre du substrat d'interconnexion 520, par exemple entre les deux pistes métalliques 524. Les pistes métalliques 525 sont dédiées aux signaux des éléments transducteurs 20 et sont reliées aux électrodes inférieures 27. Les pistes métalliques 524 et 525 s'étendent jusqu'au connecteur 522, auquel elles sont reliées.

Comme représenté dans la figure 5C, le substrat d'interconnexion 520 comprend au moins deux couches d'interconnexion, dans l'exemple une couche d'interconnexion en face avant 520D et une autre couche d'interconnexion en face arrière 520E, les pistes 525 passant sous le plot 521.

Ainsi, les pistes 525 comprennent des pistes 525A en face avant 520D du substrat d'interconnexion 520 et des pistes 525B en face arrière 520E du substrat d'interconnexion 520, les pistes 525A et 525B étant reliés entre elles dans la direction Y par des vias 527 entre la ligne de pliure **FL et** le plot 521. Au niveau du connecteur 522, les pistes 525 peuvent remonter à la première face 520D pour être connectées au connecteur 522.

Le substrat d'interconnexion 520 est partiellement recouvert au moins sur sa face avant 520D, voire sur sa face arrière 520E, d'une couche de protection 526 isolante (délimitée par des pointillés), par exemple un polymère. La couche de protection 526 laisse découverte toute la portion 520A pour que les pistes métalliques 525 puissent être reliées aux, par exemple être en contact avec les, électrodes inférieures 27 des éléments transducteurs 20. La couche de protection 526 laisse découvert le plot 521 pour que les traces métalliques 502 de reprise de masse puissent contacter ce plot 521.

Ce mode de réalisation est particulièrement avantageux, car il permet de réduire la largeur du substrat d'interconnexion 520 en comparaison avec le substrat d'interconnexion 320 de la figure 3, puisque le plot 521 s'étend transversalement uniquement jusqu'aux pistes métalliques 524.

Divers modes de réalisation et variantes ont été décrits. La personne du métier comprendra que certaines caractéristiques de ces divers modes de réalisation et variantes pourraient être combinées, et d'autres variantes apparaîtront à la personne du métier.

Enfin, la mise en œuvre pratique des modes de réalisation et variantes décrits est à la portée de la personne du métier à partir des indications fonctionnelles données ci-dessus.

## Revendications

1. Méthode de fabrication d'une sonde ultrasonore (200 ; 400), la méthode comprenant :
- la formation d'une couche diélectrique (201 ; 401) sur au moins un bord (210C, 210D) d'un empilement acoustique (210) comprenant au moins une couche piézoélectrique (211) située sur une première portion (220A ; 320A ; 520A) d'un substrat d'interconnexion (220 ; 320 ; 520), ladite première portion dudit substrat d'interconnexion étant sur une couche d'atténuation acoustique (215) et étant prolongée par une deuxième portion (220B ; 320B ; 520B) du substrat d'interconnexion repliée sur un bord (215A) de la couche d'atténuation acoustique ;
- la formation d'une structure conductrice (202 ; 402 ; 502) de reprise de masse sur des éléments transducteurs (20) formés dans l'empilement acoustique et séparés par des encoches (214) provenant de la découpe de l'empilement acoustique dans toute son épaisseur, ladite découpe s'étendant au moins jusqu'au substrat d'interconnexion ; ladite structure conductrice s'étendant sur la couche diélectrique et étant reliée à au moins un plot (221 ; 521) de contact électrique du substrat d'interconnexion ;
- la formation d'une couche de protection (204) pour recouvrir et entourer l'empilement acoustique et la structure conductrice au moins sur la hauteur des encoches.

2. Méthode de fabrication selon la revendication 1, dans laquelle la découpe s'étend dans la couche d'atténuation acoustique (215) et inclut la découpe d'au moins la première portion (220A ; 320A ; 520A) du substrat d'interconnexion (220 ; 320 ; 520).

3. Méthode de fabrication selon la revendication 1 ou 2, dans laquelle la couche diélectrique (201 ; 401) est configurée pour protéger le au moins un bord (210C, 210D) de l'empilement acoustique (210) destiné à être recouvert par la structure conductrice (202 ; 402 ; 502).

4. Méthode de fabrication selon l'une quelconque des revendications 1 à 3, dans laquelle la formation de la structure conductrice (202) comprend :
- la formation d'une couche métallique (202) sur la couche diélectrique (201), sur et autour de l'empilement acoustique (210) au moins jusqu'à être en contact avec le au moins un plot (221) ; et
- la découpe de l'empilement acoustique (210) en les éléments transducteurs (20) séparés entre eux par les encoches (214), ladite découpe incluant en outre la découpe de la couche métallique (202).

5. Méthode de fabrication selon l'une quelconque des revendications 1 à 3, dans laquelle la formation de la structure conductrice (402 ; 502) comprend :
- la découpe de l'empilement acoustique (210) en les éléments transducteurs (20) séparés entre eux par les encoches (214) ; puis
- la formation d'un réseau (402 ; 502) de traces métalliques comprenant des premières traces (402A ; 502A) sur chacun des éléments transducteurs, chaque première trace étant prolongée par une deuxième trace (402B, 402C ; 502B, 502C) s'étendant sur la couche diélectrique (401) et reliée à l'au moins un plot (221 ; 521).

6. Méthode de fabrication selon la revendication 5, dans laquelle les deuxièmes traces (402B) sont connectées à une troisième trace (402D) connectée à l'au moins un plot (221), ou les deuxièmes traces (502B) sont connectées directement à l'au moins un plot (521).

7. Méthode de fabrication selon l'une quelconque des revendications 1 à 6, comprenant en outre la formation d'une couche de blindage électromagnétique (203) recouvrant les bords de la couche d'atténuation acoustique (215) sur toute ou partie de sa hauteur, ainsi qu'au moins une partie du substrat d'interconnexion, la couche de blindage électromagnétique (203) étant une couche conductrice, par exemple sous la forme d'un adhésif en cuivre.

8. Méthode de fabrication selon la revendication 7, dans laquelle la couche de blindage électromagnétique (203) recouvre également le au moins un plot (221 ; 521).

9. Méthode de fabrication selon l'une quelconque des revendications 1 à 8, comprenant en outre la formation d'une couche (205) recouvrant et entourant la couche de protection (204) au moins sur la hauteur des encoches (214) de manière à former une lentille, la couche (205) étant par exemple une couche de silicone.

10. Méthode de fabrication selon l'une quelconque des revendications 1 à 9, dans laquelle la découpe de l'empilement acoustique (210) inclut la découpe d'une première couche métallique (216) disposée au-dessus de la couche piézoélectrique (211), par exemple sur l'empilement acoustique (210), et d'une deuxième couche métallique (217) disposée sous la couche piézoélectrique, par exemple entre l'empilement acoustique (210) et la couche d'atténuation acoustique (215), de manière à former respectivement des premières électrodes (26) et des deuxièmes électrodes (27) des éléments transducteurs (20), les premières électrodes (26) étant reliées à l'au moins un plot (221 ; 521) par l'intermédiaire de la structure conductrice (202 ; 402 ; 502).

11. Sonde ultrasonore (200 ; 400) comprenant :
- un substrat d'interconnexion (220 ; 320 ; 520) comprenant au moins une première portion (220A ; 320A ; 520A) et une deuxième portion (220B ; 320B ; 520B) reliée à la première portion ;
- un empilement acoustique (210) comprenant au moins une couche piézoélectrique (211) située sur la première portion du substrat d'interconnexion ;
- une couche diélectrique (201 ; 401) sur au moins un bord (210C, 210D) de l'empilement acoustique ;
- une couche d'atténuation acoustique (215), la première portion du substrat d'interconnexion étant entre ladite couche d'atténuation acoustique et la couche piézoélectrique, et la deuxième portion du substrat d'interconnexion étant repliée sur un bord (215A) de ladite couche d'atténuation acoustique ;
l'empilement acoustique étant divisé en plusieurs éléments transducteurs (20) séparés par des encoches (214) traversant ledit empilement acoustique dans toute son épaisseur, lesdites encoches traversant au moins la première portion du substrat d'interconnexion et s'étendant jusque dans la couche d'atténuation acoustique ;
- une structure conductrice (202 ; 402 ; 502) de reprise de masse reliée à chacun des éléments transducteurs (20), la structure conductrice s'étendant sur la couche diélectrique et étant reliée à au moins un plot (221 ; 521) de contact électrique du substrat d'interconnexion ; et
- une couche de protection (204) recouvrant et entourant l'empilement acoustique et la structure conductrice au moins sur la hauteur des encoches.

12. Sonde ultrasonore selon la revendication 11, comprenant en outre :
- une couche de blindage électromagnétique (203) recouvrant les bords de la couche d'atténuation acoustique (215) sur toute ou partie de sa hauteur, ainsi qu'au moins une partie du substrat d'interconnexion ; et/ou
- une couche (205) formant une lentille recouvrant et entourant la couche de protection (204) au moins sur la hauteur des encoches (214).

13. Méthode de fabrication selon l'une quelconque des revendications 1 à 10, ou sonde ultrasonore selon la revendication 11 ou 12, dans laquelle la couche de protection (204) :
- est un film polymère, par exemple un film polymère adhésif ; et/ou
- est métallisée au moins sur sa face qui est en regard de la structure conductrice (202 ; 402 ; 502).

14. Méthode de fabrication selon l'une quelconque des revendications 1 à 10 et 13, ou sonde ultrasonore selon l'une quelconque des revendications 11 à 13, dans laquelle le plot (221 ; 521) de contact électrique est positionné sur la deuxième portion (220B ; 320B ; 520B) du substrat d'interconnexion (220 ; 320 ; 520).

15. Méthode de fabrication selon l'une quelconque des revendications 1 à 10, 13 et 14, ou sonde ultrasonore selon l'une quelconque des revendications 11 à 14, dans laquelle:
- les encoches (214) sont en air ; et/ou
- l'empilement acoustique comprend au moins une couche d'adaptation d'impédance (212, 213) sur la couche piézoélectrique (211).
